# EUROPEAN PATENT SPECIFICATION

(11) **EP 3 125 758 B1**
(45) Date of publication and mention of the grant of the patent: **12.12.2018**
(21) Application number: 15734249.4
(22) Date of filing: 03.04.2015
(51) Int. Cl.: A61B 5/055, A61N 1/40, A61N 5/01, A61B 18/18

(54) **ACCESS GATE OR GANTRY COMPRISING AN ANTENNAS ASSEMBLY FOR THERAPY OR IMAGING**
ZUGRIFFS-GATE ODER -GANTRY MIT EINER ANTENNENANORDNUNG ZUR THERAPIE ODER BILDGEBUNG
GRILLE OU PORTIQUE D'ACCÈS COMPRENANT UN ENSEMBLE D'ANTENNES POUR UN USAGE THÉRAPEUTIQUE OU POUR L'IMAGERIE

(30) Priority: 04.04.2014 IT RM20140172
(43) Date of publication of application: 08.02.2017
(73) Proprietor: Pavoni, Pierfrancesco, 00189 Roma (IT)
(72) Inventor: Pavoni, Pierfrancesco, 00189 Roma (IT)
(74) Representative: Santi, Filippo
(86) International application number: PCT/IT2015/000095
(87) International publication number: WO 2015/151127

(56) References cited:
- US-A- 4 230 129
- US-A- 5 423 315
- US-A1- 2004 044 385
- US-A1- 2005 122 108

## Description

The present invention relates to an assembly for a therapeutical equipment or an imaging equipment, particularly for an oncological hyperthetrrmia stem, said assembly comprising antennas and an access gate or gantry. Reference is made to documents US2004/044385 A1, US4230129 A, US2005/122108 A1 and US5423315 A concerning assemblies comprising gantries and antennas. In the following, the description will be directed to an oncological hyperthermia system, but the same technical features of the invention can also be applied to other equipments, for example a computerized tomography equipment or a magnetic resonance imaging equipment, although not explicitly described.

Currently, known oncological hyperthermia equipments are provided with a fixed access gate or "gantry" and a bed, where the patient is placed, which can move both in a longitudinal direction and in a vertical direction.

Furthermore, with reference to some oncological hyperthermia equipments, an array of antennas is arranged on the gantry, with the antennas of said array of antennas coupled with the gantry in a fixed manner, and a further antenna can be positioned in the bed.

The fact that the gantry is fixed and the bed is movable also in the vertical direction causes a series of drawbacks, such as the loss of the radio frequency signal (so-called RF signal).

Furthermore, the antennas of the array of antennas do not allow to adapt the equipment to the size of the patient subjected to a treatment, since they are coupled with the gantry in a fixed manner.

Object of the present invention is to provide a gantry configured to perform a movement in a vertical direction, so as to prevent the bed from moving in the same direction. In this way, the gantry can be lifted and lowered if necessary and positioned at a predetermined height depending on the size of the patient.

The invention is defined in the appended set of claims.

With the technical solution proposed according to the present invention, the following advantages are obtained:
- a lower loss of the RF signal;
- a lower mechanical stress of an RF cable;
- a simple and fast manoeuvre to extract the bed, on which the patient lays, from the gantry in any situation, especially useful in emergency situations;
- a greater comfort for the patient and for the operator who has to access to the gantry, the bed and the patient.

In particular, with reference to the loss of an RF signal, it is known as a loss of the RF signal is directly proportional to the length of the RF cable which carries the RF signal from the RF amplifiers, generally positioned in a dedicated room (different from the one where the gantry is positioned), to the antennas of which the gantry is provided and to the antenna positioned in the bed.

With the technical solution according to the invention, the fact that the gantry moves in a vertical direction reduces the length of the RF cables to the minimum.

If the antenna is contained in a bed which can move vertically, laterally and longitudinally, the length of the RF cable connected to this antenna has to be such to allow the bed these three movements. Instead, if the antenna is contained in a bed that can only move longitudinally, the length of the RF cable has to be such to ensure only this movement.

It must also take into consideration that, for reasons of transmission and control of an RF signal in phase and amplitude, the length of the RF cables that are connected to each antenna has to be the same. Therefore, the length of the RF cable associated with the antenna farthest from the position of the amplifiers determines the length of all other RF cables connected to the remaining antennas.

In the case where the bed moves only in a vertical direction, the gantry has to be configured to perform a longitudinal movement to allow the patient to be placed on a bed.

However, in this case, the length of each RF cable becomes significant and therefore each RF signal is subject to a significant loss of signal.

With regard to the mechanical stress of an RF cable, it is known that it determines over time a wear of the RF cable which can even lead to a break of the same.

With the technical solution according to the invention, the mechanical stresses at which the RF cables are subjected are minimized, due to the choice of moving the gantry in a vertical direction rather than the bed in a vertical and horizontal direction and/or the gantry in a horizontal direction.

Furthermore, with the technical solution according to the invention, it is possible to extract the bed from the gantry in a simple and quick way.

This is particularly advantageous in emergency situations, where the time available to an operator to extract the bed from the gantry is one minute.

With the present invention, it is possible to ensure a simple and rapid manoeuvre to extract the bed from the gantry by means of a bed capable of performing only a longitudinal movement, in particular manual.

In this way, in cases of emergency, it is possible to move the bed away from the gantry, thanks to just one action from an operator, so that the bed is at a predetermined distance from said gantry.

Unlike to the known equipments, where the gantry is fixed and the bed is movable in a plurality of directions, in the present invention, the gantry moves in a vertical direction and the bed in a longitudinal direction, and then the bed does not require remote control to obtain a motorized movement in the vertical and longitudinal directions in order to extract the bed from the gantry.

Finally, it is possible to obtain a greater comfort for the patient as the gantry can be lifted so as to allow the patient to be placed on the bed, when the latter is external to the gantry, and, once the patient is placed on the bed, to move said bed in the longitudinal direction of a predetermined distance until it is inside the gantry, preferably in a central position, so that the gantry is partially overlapped with the bed itself. At this point, it is possible to lower the gantry and correctly position the antennas of the gantry with respect to the size of the patient placed on the bed.

Thanks to the vertical movement of the gantry and the horizontal movement of the bed, the operator has the possibility to freely access the gantry, the bed and the patient, without the impediments linked to a limitation of movements that would be present if the gantry and bed were fixed or if the gantry was fixed and the bed was movable vertically or if the gantry was movable longitudinally and the bed was movable only vertically.

It is therefore specific object ot the present invention an access gate or gantry - antennas assembly for a therapeutical equipment or for an imaging equipment, particularly for an oncological hyperthermia system, where said assembly comprises an access gate or gantry, an array of first antennas coupled with said gantry, a bed movable at least longitudinally with respect to said gantry, and at least one second antenna provided in said bed. In particular, said assembly comprises lifting and lowering means for lifting and lowering said access gate or gantry.

In a first alternative, said lifting and lowering means are comprised of hydraulic means or pneumatic means or electromechanical means.

In a second alternative, said lifting and lowering means are comprised of mechanical means, such as gears and racks or pulleys and toothed belts.

Each of said first antennas can be provided with translation means for translating with respect to the inner part of said gantry, and rotation means for rotating with respect to the gantry itself.

In particular, each first antenna can comprise a first shell, coupled with said gantry, and a second shell, coupled with said first shell and arranged within said first shell. Said translation means can be configured to translate said second shell in a first direction of a predetermined distance, and said rotation means can be configured to rotate said first shell of a predetermined angle. Said second shell can be coupled with said first shell so that, when said first shell rotates, said second shell rotates; the first shell of each first antenna can be coupled with said gantry by respective rotation means.

Said translation means can comprise at least one electromechanical actuator and a plurality of linear guides, and said rotation means can comprise a turntable.

Furthermore, each first antenna can comprise a third shell to facilitate the translation of said second shell, where said third shell is arranged within said second shell and dimensioned such that said second shell is partially overlapped with said first shell. Each linear guide can comprise a first guide element, arranged on said second shell, and a second guide element, arranged on said third shell, where said first guide element and said second guide element are coupled together to allow the sliding of said first guide element with respect to said second guide element.

In particular, four linear guides are provided.

Each second shell can have four sides and each linear guide can be arranged on a respective side in such a way that the projection of it on the opposite side falls on a first portion of said opposite side, different from a second portion of said opposite side on which the respective linear guide is arranged.

With reference to the bed, said bed can comprise at least one seat for housing said at least one second antenna.

In a first alternative, said seat can be fixed and said second antenna can be fixed.

In a second alternative, said bed can be provided with rotation means for rotating said seat, and said second antenna can be coupled with said seat so that, when said seat rotates, said second antenna rotates.

Furthermore, said bed can comprise a plurality of second antennas and a respective seat for each of said second antennas.

It is preferable that said bed has a substantially T-shaped section.

With reference to the array of first antennas, said array of first antennas can preferably comprise three first antennas.

Said three first antennas can be arranged at the vertices of a triangle, where said triangle is preferably an isosceles or equilateral triangle.

Advantageously, said gantry can comprise in its interior at least one ventilation device to generate an air flow, positioned in such a way to direct said air flow toward a patient placed on said bed.

The present invention will be now described, for illustrative, but not limitative purposes, according to its embodiment, making particular reference to the enclosed figures, wherein:
Figure 1A is a perspective view of an of oncological hyperthermia system comprising an access gate or gantry - antennas assembly, according to the invention, where said assembly comprises an access gate or gantry and a bed;
Figure 1B is a side view of the assembly of Figure 1A;
Figure 1C is a top view of the bed;
Figure 2 is a schematic front view of an assembly according to the invention, without bed, which shows the first antennas arranged on the gantry and configured to translate and a second antenna positioned in the bed;
Figures 3A and 3B show respectively the first antennas of the gantry in a first position, in which they are not in contact with the body of a patient placed on the bed, and in a second position, in which they are translated to be positioned substantially in contact with a respective portion of the body of the patient;
Figure 4 is a schematic view of the assembly that shows the first antennas of the gantry and the second antenna of the bed rotated by 90° about the respective axis;
Figure 5A is a schematic front view of the assembly, without bed, which shows the first antennas of the gantry translated and rotated by 90° and the second antenna of the bed rotated by 90°;
Figure 5B is a schematic front view of the assembly, without bed, which shows only one of the first antennas of the gantry rotated by 90° and the second antenna of the bed rotated by 90°;
Figure 6A schematically shows, with reference to a first antenna of the gantry, a frame portion of a first shell, a frame portion of a second shell, arranged within said first shell, a frame portion of a third shell, arranged within said second shell, and the position of the translation means for translating said first antenna, where said translation means comprises an electromechanical actuator and a plurality of linear guides, and of the rotation means for rotating said first antenna, where said rotation means comprises a turntable;
Figure 6B shows a first antenna of the gantry from which a portion has been removed to make the three shells visible as well as the electromechanical actuator and a linear guide;
Figure 7A is a schematic side view showing the position of the electromechanical actuator and the linear guides with respect to the second shell of a first antenna;
Figure 7B is a schematic front view showing the position of the electromechanical actuator and the linear guides with respect to the second shell and the third shell of a first antenna.

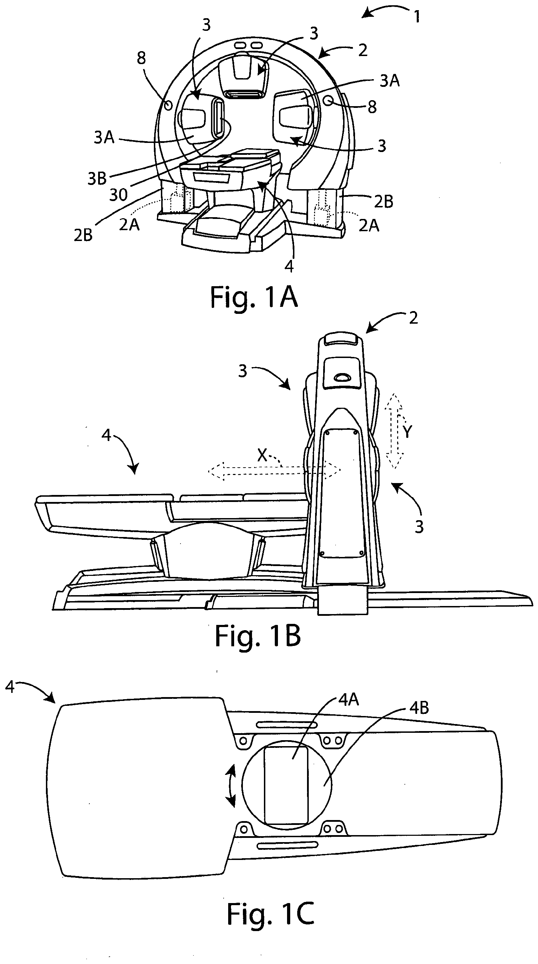

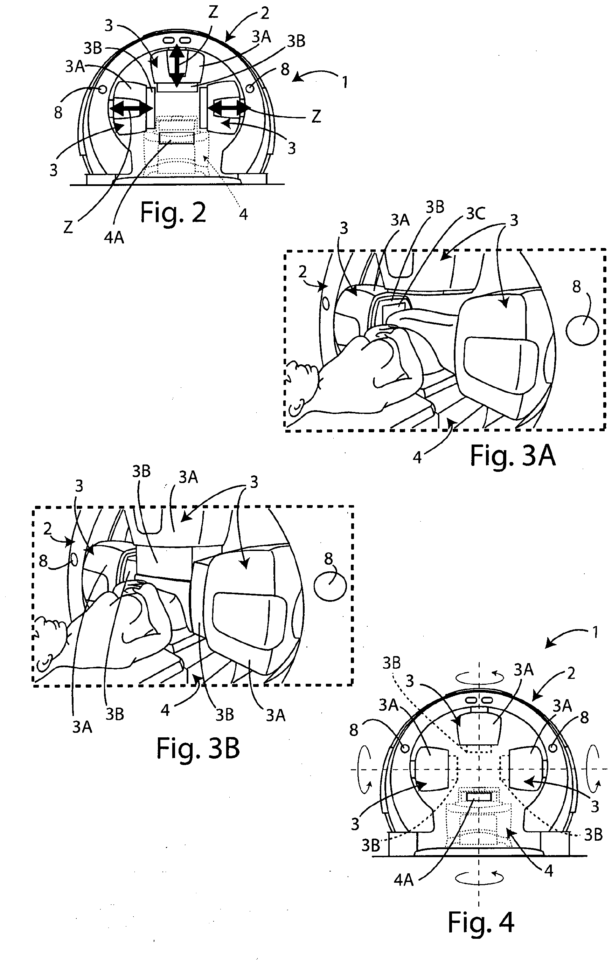

With particular reference to Figures 1A-1C and 2, an oncological hyperthermia system according to the invention, indicated by the number reference 1, is shown.

Said system comprises a gantry 2, an array of first antennas 3, coupled with said gantry 2, and a bed 4, on which a patient is placed, where said bed has a second antenna 4A positioned inside.

Although not shown in the figures, it is possible to provide a plurality of second antennas arranged within said bed, without departing from the scope of the invention.

In particular, the gantry 2 is configured to perform a movement in a vertical direction so as to be lifted and lowered (see the double arrow Y shown in Figure 1B), and the bed 4 is configured to perform a longitudinal movement so as to be movable between a first position, in which it is external to said gantry 2, to a second position in which said gantry 2 is partially overlapped with said bed 4 so that the patient can be subjected to the specific treatment that is required (see the double arrow X shown in Figure 1B), and vice versa.

With reference to the gantry 2, said gantry 2 performs said movement in a vertical direction through lifting and lowering means 2A for lifting and lowering said gantry.

In particular, said gantry 2 has substantially the form of an open arch having two ends on each of which said lifting and lowering means 2A act, where said lifting and lowering means are positioned inside a casing 2B (Fig. 1A).

Said lifting and lowering means 2A can be constituted by means of pneumatic or hydraulic or electromechanical lifting and lowering means or mechanical lifting and lowering means, such as rack and gear, pulleys and toothed belts, etc.

In the example that is described, said lifting and lowering means 2A are of an electromechanical type.

With reference to the bed 4, said bed 4 has a longitudinal section substantially T-shaped and is provided with a seat for housing said second antenna 4B 4A, arranged in a central position (Fig. 1C).

In case of a plurality of second antennas arranged in the bed, said bed is provided with a seat for housing a respective second antenna.

With reference to the first antennas 3, said first antennas 3 are configured to perform a translational movement and a rotational movement.

Each of said first antenna 3 is configured to translate in a first direction of a predetermined distance, regardless of the translation of the other first antennas 3, and to rotate by a predetermined angle, regardless of the rotation angle of the other first antennas 3.

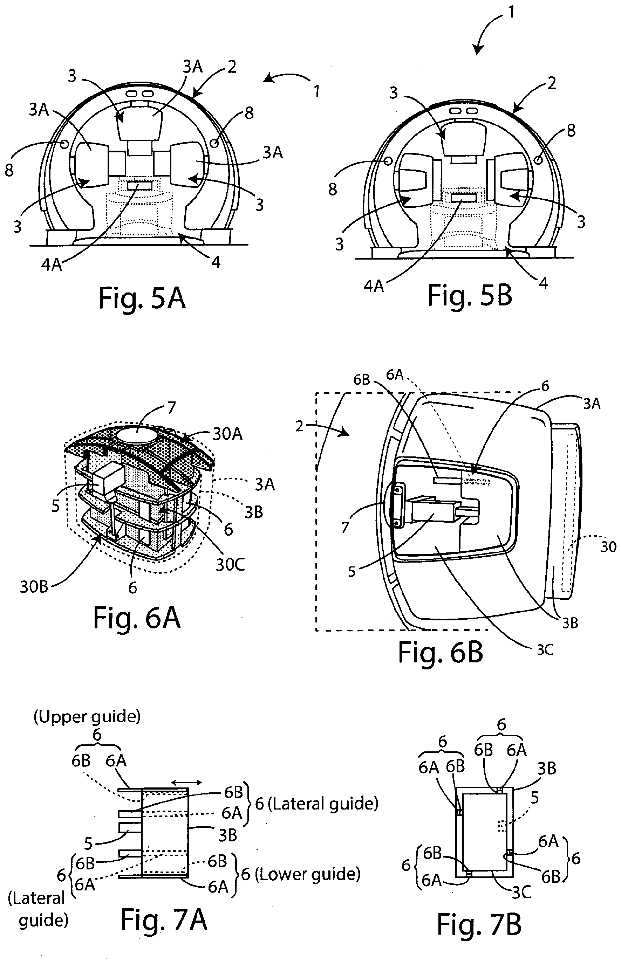

Each of said first antenna 3 is coupled with the gantry 2 by means of a coupling element 7 (described with reference to figure 6A), and comprises a first shell 3A, a second shell 3B, arranged within said first shell 3A, and transmission means 30 for emitting an RF signal, arranged inside said second shell 3B. Alternatively, said transmission means may be replaced by transceiver means for emitting and receiving an RF signal.

In the example that is described, the number of said first antenna 3 is equal to three. However, although not shown in the figures, said array of first antennas may comprise a number of antennas greater than or equal to two.

Said first three antennas are arranged on the gantry 2 to the vertices of a triangle. In particular, said first antennas 3 are arranged at the vertices of an equilateral triangle. However, although not shown in the figures, said first antenna 3 can be arranged at the vertices of an isosceles triangle.

Furthermore, in the example being described, each of said first antenna 3 has two sides having a first length, and two sides having a second length, wherein said second length is lower than said first length.

With reference to the second antenna 4A, said second antenna 4A is configured to perform a rotation movement.

Said second antenna 4A may comprise transmission means for emitting an RF signal or transceiver means for emitting and receiving an RF signal.

In particular, the second antenna 4A is configured to rotate by a predetermined angle, regardless of the angle of rotation of each of the first antennas 3 arranged on the gantry 2.

To this end, said bed 4 is provided with rotation means (not shown) to rotate the seat 4B in which said second antenna 4A is housed, and said second antenna is coupled with said seat 4B so that, when said seat 4A rotates, said second antenna 4B rotates accordingly (fig. 1C).

Although not shown in the figures, said bed may be devoid of rotation means, so that the seat is not configured to rotate and the second antenna is fixed.

Furthermore, it is preferable that said second antenna 4A is not capable of performing a translation movement.

Furthermore, in the example that is described, said second antenna 4A has two sides having a first length, and two sides having a second length, wherein said second length is lower than said first length.

With particular reference to figure 2, it is clear as said first antennas 3 can perform a translation movement in a first direction, perpendicular to its opening plane, to and from the patient, so as to be moved closer or away with respect to/from the body of the patient (see the double arrow Z for each first antenna 3).

In fact, each of said first antennas 3 is configured to translate from a first position, in which it is at a predetermined distance from the body of the patient placed on the bed, to a second position, in which it is moved forward in a first direction by a predetermined distance to be positioned substantially in contact with a respective portion of the body of the patient, and vice versa (Figures 3A and 3B).

In particular, it is the second shell 3B (in which said transmission means 30 are arranged) which is configured to translate in such a way that is moved closer or away to/from the body of the patient disposed on the bed.

In other words, the second shell 3B is configured to move from a first position, in which it is in a retracted position, inside the first shell 3A, to a second position, in which it is in an extended position with respect to said first shell 3A, and vice versa.

Furthermore, said first shell 3A is configured to rotate with respect to the gantry 2, and said second shell 3B is coupled with said first shell 3A so that, when said first shell 3A rotates, said second shell 3B rotates accordingly.

With reference to the second antenna 4A arranged in the bed 4, as previously said, it is configured to rotate about its axis by means of the seat 4B of which the bed 4 is provided.

Figure 4 shows the first antennas 3 arranged on the gantry 2 and the second antenna 4A arranged in the bed 4 rotated by 90° about the respective axis.

Figure 5A shows the first antennas 3 arranged on the gantry 2 translated and rotated by 90° and the second antenna 4A arranged in the bed 4 rotated by 90°.

The translation and rotation of said first antenna 3 and the rotation of said second antenna offer the possibility to treat the patient's anatomical regions of smaller size than the size of the trunk of the patient, such as the limbs.

Figure 5B shows all of the first antennas 3 translated, of which only one of them is rotated by 90°, and the second antenna 4A rotated by 90°. In the example that is described, the first antenna 3 rotated by 90° is arranged at the upper vertex of the triangle.

The rotation of the first antennas 3 and that of the second antenna element 4A is a element of complete innovation with respect to the known equipments.

Figures 6A and 6B show the presence of a third shell 3C, arranged within the second shell 3B of a first antenna 3, as well as said translation means and said rotation means.

With particular reference to figure 6A, there is shown a frame portion 30A of the first shell 3A of a first antenna 3, a frame portion 30B of the second shell 3B of the same first antenna, a frame portion 30C of said third shell 3C. Each portion is represented by a respective dotted pattern.

From figure 6A itself the design elements that allow the translation and rotation of said first antenna 3 are visible.

Said design elements comprise translation means for translating said first antenna 3 with respect to the gantry 2 so that it can be moved closer and away with respect to the patient and rotation means for rotating said first antenna 3 with respect to the inner part of the gantry.

Said translation means comprise at least one electromechanical actuator 5 and a plurality of linear guides 6. In the example that is described, said translation means comprise an electromechanical actuator element 5, four linear guides 6 (of which only two are shown in figure 6A).

Said rotation means comprise a turntable 7 for rotating said first antenna about its axis. The rotation of said turntable may be manual or motorized.

In particular, in addition to allowing the rotation of a first antenna with respect to the inner part of the gantry, said rotation means constitute the coupling element, which allows the coupling of a first antenna 3 with the gantry 2.

The fact that each of said first antenna 3 is coupled with the gantry 2 by means of respective rotation means allows the rotation of a first antenna 3 independently of the other first antennas 3.

Furthermore, figure 6A shows a frame portion 30C of a third shell 3C, arranged within said second shell 3B.

It is preferable that each first antenna 3 comprises said third shell 3C to facilitate the translation of said second shell 3B (comprising said transmission means 30) to be moved closer or away to/from the patient.

Figure 6B shows a first antenna 3 from which a portion of the first shell 3A has been removed to make visible the second shell 3B, the third shell 3C, the electromechanical actuator 5 and a linear guide 6.

Said third shell 3C is dimensioned so that said second shell 3B is partially overlapped with it, both when the latter is not translated and when it is translated.

As shown in figure 6B, said second shell 3B is translated and is partially overlapped with it.

With reference to the electromechanical actuator 5, the design choice of positioning said electromechanical actuator 5 to the center of one of the two longer sides of said first antenna 3 together with the position of the linear guides 6 (described below) allows to obtain an optimal stability in the linear translation of the first antenna 3 itself, even when said first antenna 3 is rotated by a predetermined angle about its axis.

In the example that is described, said electromechanical actuator 5 is coupled with said third shell 3C and is arranged so as to act on said second shell 3B to allow the translation of the latter.

With reference to the four linear guides 6, each of them comprises a first guide element 6A (which can be for example a male element or a female element), arranged on said second shell 3B, and a second guide element 6B (which may be a female or a male element), arranged on said third shell 3C, coupled together to allow the sliding of said first guide element 6A with respect to said second guide element 6B.

In particular, two linear guides 6 are respectively arranged on a longer side of said second shell 3B and the remaining two linear guides 6 are respectively arranged on a shorther side of said second shell 3B.

Figures 7A and 7B show the position of the linear guides 6 for guiding the translation of the second shell 3B of a first antenna 3.

From such figures it is clear that two linear guides 6 are lateral guides, as arranged respectively on the right and on the left side of said second shell 3B (i.e. the longer sides), and the other two linear guides 6 respectively constitute a guide linear upper and a lower linear guide, as arranged respectively on the upper side and on the lower side of the second shell 3B itself (i.e. the shorter sides).

In particular, the two lateral linear guides 6 are arranged respectively on the left and right side of said second shell 3B in such a way that the projection of each of them on the opposite side falls on a first portion of said opposite side, different from a second portion of said opposite side on which the respective linear guide is arranged.

Moreover, the two upper and lower linear guides 6 are arranged respectively on the upper and lower side of said second shell 3B in such a way that the projection of each of them on the opposite side falls on a portion of said opposite side, different from that on which the linear guide of said opposite side is arranged.

Said four linear guides 6 are arranged in such a way that they ensure the movement of said second shell 3B (to be moved closer and away to/from the patient's body), without this being subject to bending and/or rotating and/or tilting during this movement.

According to the invention, advantageously, said gantry 2 comprises in its interior at least one ventilation device 8 for generating an air flow, where said ventilation device is placed in such a way to direct such air flow toward a patient placed on said bed 4 (fig. 1a, 2.3A, 3B, 4,5A, 5B).

In particular, in the embodiment being described, said gantry comprises in its interior two ventilation devices 8. Said ventilation devices 8 are arranged on a same first plane transverse to said gantry. However, any number of ventilation devices can be provided within the gantry, without departing from the scope of the invention.

For example it is possible to provide two further ventilation devices 8 arranged on a second plane transverse to said gantry 2, parallel to said first plane.

The present invention has been described for illustrative, but not limitative purposes, with particular reference to a preferred embodiment, but it is to be understood that variations and/or modifications can be carried out by a skilled in the art without departing from the scope thereof, as defined according to enclosed claims.

## Claims

1. Assembly for a therapeutical equipment or for an imaging equipment, particularly for an oncological hyperthermia system, said assembly comprising an access gate or gantry (2), an array of first antennas (3) coupled with said gantry (2), a bed (4), said bed being at least longitudinally movable with respect to said gantry (2), and at least one second antenna provided in said bed, said assembly comprising lifting and lowering means (2A) for lifting and lowering said access gate or gantry (2).

2. Assembly according to the preceding claim, **characterized in that** each of said first antennas (3) is provided with translation means (5, 6) for translating with respect to the inner part of said gantry (2), and rotation means for rotating with respect to the gantry (2) itself.

3. Assembly according to the preceding claim, **characterized in that**
each first antenna (3) comprises a first shell (3A), coupled with said gantry (2), and a second shell (3B), coupled with said first shell (3A) and arranged within said first shell (3A),
**in that**
said translation means (5, 6) are configured to translate said second shell (3B) in a first direction of a predetermined distance, and
**in that**
said rotation means (7) are configured to rotate said first shell (3A) of a predetermined angle; said second shell (3B) being coupled with said first shell (3A) so that, when said first shell (3A) rotates, said second shell (3B) rotates; the first shell (3A) of each first antenna (3) being coupled with said gantry (2) by respective rotation means (7).

4. Assembly according to claim 2 or 3, **characterized in that** said translation means (5, 6) comprises at least one electromechanical actuator (5) and a plurality of linear guides (6), and **in that** said rotation means (7) comprises a turntable (7).

5. Assembly according to the preceding claim referred back to claim 3, **characterized in that** each first antenna (3) comprises a third shell (3C) to facilitate the translation of said second shell (3B), said third shell (3C) being arranged within said second shell (3B) and dimensioned such that said second shell (3B) is partially overlapped with said first shell (3A),
**in that**
each linear guide (6) comprises a first guide element (6A), arranged on said second shell (3B), and a second guide element (6B), arranged on said third shell (3C), where said first guide element (6A) and said second guide element (6B) are coupled together to allow the sliding of said first guide element (6A) with respect to said second guide element (6B).

6. Assembly according to claim 4 or 5, **characterized in that** four linear guides (6) are provided.

7. Assembly according to claim 3 and any one of claims 4-6, **characterized in that** each second shell (3B) has four sides and **in that** each linear guide (6) is arranged on a respective side in such a way that the projection of it on the opposite side falls on a first portion of said opposite side, different from a second portion of said opposite side on which the respective linear guide (6) is arranged.

8. Assembly according to any one of claims 1-7, **characterized in that** said bed (4) comprises at least one seat (4B) for housing said at least one second antenna (4A) and **in that** said seat is fixed and said second antenna is fixed.

9. Assembly according to any one of claims 1-7, **characterized in that** said bed (4) comprises at least one seat (4B) for housing said at least one second antenna (4A), **in that** said bed is provided with rotation means for rotating said seat (4B), and **in that** said second antenna (4A) is coupled with said seat (4B) so that, when said seat (4B) rotates, said second antenna (4A) rotates.

10. Assembly according to any one of the preceding claims, **characterized in that** said bed (4) comprises a plurality of second antennas (4A); said bed (4) comprising a respective seat for each second antenna.

11. Assembly according to any one of the preceding claims, **characterized in that** said bed (4) has a substantially T-shaped section.

12. Assembly according to any one of the preceding claims, **characterized in that** said array of first antennas (3) comprises three first

13. Assembly according to the preceding claim, **characterized in that** said three first antennas (3) are arranged at the vertices of a triangle; said triangle preferably being an isosceles or equilateral triangle.

14. Assembly according to any one of claims 1-13, **characterized in that** said lifting and lowering means (2A) comprise hydraulic means, pneumatic means or electromechanical means.

15. Assembly according to any one of claims 1-13, **characterized in that** said lifting and lowering means (2A) comprise mechanical means, such as gears and racks or pulleys and toothed belts.

16. Assembly according to any one of the preceding claims, **characterized in that** said gantry (2) comprises in its interior at least one ventilation device (8) to generate an air flow, said ventilation device being positioned in such a way to direct said air flow toward a patient placed on said bed (4).

## Patentansprüche

1. Anordnung für eine therapeutische Einrichtung oder für eine bildgebende Einrichtung, insbesondere für ein onkologisches Hyperthermiesystem, wobei die Anordnung ein(e) Zugriffsgate- oder -Gantry (2), eine mit der Gantry (2) gekoppelte Gruppe von ersten Antennen (3), ein Bett (4), wobei das Bett bezüglich der Gantry (2) mindestens längswärts beweglich ist, und mindestens eine zweite Antenne, vorgesehen in dem Bett, umfasst, wobei die Anordnung Hebe- und Senkmittel (2A) zum Heben und Senken des Zugriffsgates oder der Gantry (2) umfasst.

2. Anordnung nach dem vorstehenden Anspruch, **dadurch gekennzeichnet, dass** jede von den ersten Antennen (3) mit Verschiebemitteln (5, 6) zum Verschieben bezüglich des inneren Teils der Gantry (2) und Drehmitteln zum Drehen bezüglich der Gantry (2) selbst versehen ist.

3. Anordnung nach dem vorstehenden Anspruch, **dadurch gekennzeichnet,**
**dass** jede erste Antenne (3) eine erste Schale (3A), gekoppelt mit der Gantry (2), und eine zweite Schale (3B), gekoppelt mit der ersten Schale (3A) und angeordnet in der ersten Schale (3A), umfasst,
**dass** die Verschiebemittel (5, 6) zum Verschieben der zweiten Schale (3B) in eine erste Richtung einer vorbestimmten Strecke ausgelegt sind, und
**dass** die Drehmittel (7) zum Drehen der ersten Schale (3A) um einen vorbestimmten Winkel ausgelegt sind; wobei die zweite Schale (3B) so mit der ersten Schale (3A) gekoppelt ist, dass, wenn die erste Schale (3A) sich dreht, sich die zweite Schale (3B) dreht; wobei die erste Schale (3A) von jeder ersten Antenne (3) mit der Gantry (2) durch jeweilige Drehmittel (7) gekoppelt ist.

4. Anordnung nach Anspruch 2 oder 3, **dadurch gekennzeichnet, dass** die Verschiebemittel (5, 6) mindestens einen elektromechanischen Betätiger (5) und eine Vielzahl von Linearführungen (6) umfassen, und dass das Drehmittel (7) eine Drehscheibe (7) umfasst.

5. Anordnung nach dem vorstehenden Anspruch, zurückbezogen auf Anspruch 3, **dadurch gekennzeichnet, dass**
jede erste Antenne (3) eine dritte Schale (3C) zum Erleichtern der Verschiebung der zweiten Schale (3B) umfasst, wobei die dritte Schale (3C) in der zweiten Schale (3B) angeordnet ist und so bemessen ist, dass die zweite Schale (3B) teilweise mit der ersten Schale (3A) überlappt,
dass jede Linearführung (6) ein erstes Führungselement (6A), das auf der zweiten Schale (3B) angeordnet ist, und ein zweites Führungselement (6B), das auf der dritten Schale (3C) angeordnet ist, umfasst, wobei das erste Führungselement (6A) und das zweite Führungselement (6B) zusammen gekoppelt sind, um das Gleiten des ersten Führungselements (6A) bezüglich des zweiten Führungselements (6B) zu erlauben.

6. Anordnung nach Anspruch 4 oder 5, **dadurch gekennzeichnet, dass** vier Linearführungen (6) vorgesehen sind.

7. Anordnung nach Anspruch 3 und einem von Ansprüchen 4-6, **dadurch gekennzeichnet, dass** jede zweite Schale (3B) vier Seiten aufweist und dass jede Linearführung (6) an einer jeweiligen Seite solchermaßen angeordnet ist, dass die Projektion davon auf die entgegengesetzte Seite an einen ersten Abschnitt der entgegengesetzten Seite fällt, die von einem zweiten Abschnitt der entgegengesetzten Seite, an der die jeweilige Linearführung (6) angeordnet ist, verschieden ist.

8. Anordnung nach einem der Ansprüche 1-7, **dadurch gekennzeichnet, dass** das Bett (4) mindestens einen Sitz (4B) zum Beherbergen der mindestens einen zweiten Antenne (4A) umfasst und dass der Sitz befestigt ist und die zweite Antenne befestigt ist.

9. Anordnung nach einem der Ansprüche 1-7, **dadurch gekennzeichnet, dass** das Bett (4) mindestens einen Sitz (4B) zum Beherbergen der mindestens einen zweiten Antenne (4A) umfasst, dass das Bett mit Drehmitteln zum Drehen des Sitzes (4B) versehen ist, und dass die zweite Antenne (4A) mit dem Sitz (4B) so gekoppelt ist, dass wenn der Sitz (4B) sich dreht, sich die zweite Antenne (4A) dreht.

10. Anordnung nach einem der vorstehenden Ansprüche, **dadurch gekennzeichnet, dass** das Bett (4) eine Vielzahl von zweiten Antennen (4A) umfasst; wobei das Bett (4) einen jeweiligen Sitz für jede zweite Antenne umfasst.

11. Anordnung nach einem der vorstehenden Ansprüche, **dadurch gekennzeichnet, dass** das Bett (4) einen im Wesentlichen T-förmigen Abschnitt aufweist.

12. Anordnung nach einem der vorstehenden Ansprüche, **dadurch gekennzeichnet, dass** die Gruppe von ersten Antennen (3) drei erste Antennen (3) umfasst.

13. Anordnung nach dem vorstehenden Anspruch, **dadurch gekennzeichnet, dass** die drei ersten Antennen (3) an den Scheitelpunkten eines Dreiecks angeordnet sind; wobei das Dreieck vorzugsweise ein gleichschenkliges oder gleichseitiges Dreieck ist.

14. Anordnung nach einem der Ansprüche 1-13, **dadurch gekennzeichnet, dass** die Hebe- und Senkmittel (2A) hydraulische Mittel, pneumatische Mittel oder elektromechanische Mittel umfassen.

15. Anordnung nach einem der Ansprüche 1-13, **dadurch gekennzeichnet, dass** die Hebe- und Senkmittel (2A) mechanische Mittel umfassen, wie Zahnräder und Zahnstangen oder Rollen und Zahngurte.

16. Anordnung nach einem der vorstehenden Ansprüche, **dadurch gekennzeichnet, dass** die Gantry (2) in ihrem Inneren mindestens eine Belüftungsvorrichtung (8) zum Erzeugen eines Luftstroms umfasst, wobei die Belüftungsvorrichtung solchermaßen positioniert ist, dass der Luftstrom zu einem Patienten gerichtet ist, der sich auf dem Bett (4) befindet.

## Revendications

1. Assemblage pour un équipement thérapeutique ou pour un équipement d'imagerie, en particulier pour un système d'hyperthermie oncologique, ledit assemblage comprenant une porte ou un portique d'accès (2), un réseau de premières antennes (3) couplé audit portique (2), un lit (4), ledit lit étant mobile longitudinalement par rapport audit portique (2), et au moins une deuxième antenne fournie dans ledit lit, ledit assemblage comprenant des moyens de levage et d'abaissement (2A) pour lever ou abaisser ladite porte ou ledit portique d'accès (2).

2. Assemblage selon la revendication précédente, **caractérisé en ce que** chacune desdites premières antennes (3) est pourvue de moyens de translation (5, 6) pour une translation par rapport à la partie intérieure dudit portique (2), et de moyens de rotation par rapport au portique (2) lui-même.

3. Assemblage selon la revendication précédente, **caractérisé en ce que** chaque première antenne (3) comprend une première coque (3A), couplée audit portique (2), et une deuxième coque (3B), couplée à ladite première coque (3A) et arrangée à l'intérieur de ladite première coque (3A),
**en ce que**
lesdits moyens de translation (5, 6) sont configurés pour translater ladite deuxième coque (3B) dans une première direction d'une distance prédéterminée, et
**en ce que**
lesdits moyens de rotation (7) sont configurés pour faire tourner ladite première coque (3A) d'un angle prédéterminé ; ladite deuxième coque (3B) étant couplée à ladite première coque (3A) de sorte que, lorsque ladite première coque (3A) tourne, ladite deuxième coque (3B) tourne ; la première coque (3A) de chaque première antenne (3) étant couplée audit portique (2) par des moyens de rotation (7) respectifs.

4. Assemblage selon la revendication 2 ou 3, **caractérisé en ce que** lesdits moyens de translation (5, 6) comprennent au moins un actuateur électromécanique (5) et une pluralité de guides linéaires (6), et **en ce que** lesdits moyens de rotation (7) comprennent une plaque tournante (7).

5. Assemblage selon la revendication précédente en ce qu'elle se réfère à la revendication 3, **caractérisé en ce que**
chaque première antenne (3) comprend une troisième coque (3C) pour faciliter la translation de ladite deuxième coque (3B), ladite troisième coque (3C) étant arrangée à l'intérieur de ladite deuxième coque (3B) et dimensionnée de sorte que ladite deuxième coque (3B) est partiellement recouverte par ladite première coque (3A),
**en ce que**
chaque guide linéaire (6) comprend un premier élément de guide (6A), arrangé sur ladite deuxième coque (3B), et un deuxième élément de guide (6B), arrangé sur ladite troisième coque (3C), où ledit premier élément de guide (6A) et ledit deuxième élément de guide (6B) sont couplés ensemble pour permettre le glissement dudit premier élément de guide (6A) par rapport audit deuxième élément de guide (6B).

6. Assemblage selon la revendication 4 ou 5, **caractérisé en ce que** quatre guides linéaires (6) sont fournis.

7. Assemblage selon la revendication 3 et l'une quelconque des revendications 4 à 6, **caractérisée en ce que** chaque deuxième coque (3B) a quatre côtés et **en ce que** chaque guide linéaire (6) est arrangé sur un côté respectif de telle sorte que sa projection sur le côté opposé tombe sur une première portion dudit côté opposé, différente d'une deuxième portion dudit côté opposé sur laquelle le guide linéaire (6) respectif est arrangé.

8. Assemblage selon l'une quelconque des revendications 1 à 7, **caractérisé en ce que** ledit lit (4) comprend au moins un siège (4B) pour loger ladite au moins une deuxième antenne (4A) et **en ce que** ledit siège est fixé et ladite deuxième antenne est fixée.

9. Assemblage selon l'une quelconque des revendications 1 à 7, **caractérisé en ce que** ledit lit (4) comprend au moins un siège (4B) pour loger ladite au moins une deuxième antenne (4A), **en ce que** ledit lit est pourvu de moyens de rotation pour faire tourner ledit siège (4B), et **en ce que** ladite deuxième antenne (4A) est couplée audit siège (4B) de sorte que, lorsque ledit siège (4B) tourne, ladite deuxième antenne (4A) tourne.

10. Assemblage selon l'une quelconque des revendications précédentes, **caractérisé en ce que** ledit lit (4) comprend une pluralité de deuxièmes antennes (4A) ; ledit lit (4) comprenant un siège respectif pour chaque deuxième antenne.

11. Assemblage selon l'une quelconque des revendications précédentes, **caractérisé en ce que** ledit lit (4) a une section essentiellement en forme de T.

12. Assemblage selon l'une quelconque des revendications précédentes, **caractérisé en ce que** ledit réseau de premières antennes (3) comprend trois premières antennes (3).

13. Assemblage selon la revendication précédente, **caractérisé en ce que** lesdites trois premières antennes (3) sont arrangées aux sommets d'un triangle ; ledit triangle étant de préférence un triangle isocèle ou équilatéral.

14. Assemblage selon l'une quelconque des revendications 1 à 13, **caractérisé en ce que** lesdits moyens de levage et d'abaissement (2A) comprennent des moyens hydrauliques, des moyens pneumatiques ou des moyens électromécaniques.

15. Assemblage selon l'une quelconque des revendications 1 à 13, **caractérisé en ce que** lesdits moyens de levage et d'abaissement (2A) comprennent des moyens mécaniques, tels que des engrenages et des crémaillères ou des poulies et des courroies crantées.

16. Assemblage selon l'une quelconque des revendications précédentes, **caractérisé en ce que** ledit portique (2) comprend en son sein au moins un dispositif de ventilation (8) pour produire un flux d'air, ledit dispositif de ventilation étant positionné de manière à diriger ledit flux d'air vers un patient placé sur ledit lit (4).
